# EUROPEAN PATENT SPECIFICATION

(11) **EP 1 334 367 B1**
(45) Date of publication and mention of the grant of the patent: **07.06.2006**
(21) Application number: 01981868.1
(22) Date of filing: 12.10.2001
(51) Int. Cl.: G01N 33/74

(54) **A METHOD OF DETERMINING THE INITIAL DOSE OF VITAMIN D COMPOUNDS**
VERFAHREN ZUR BESTIMMUNG DER ANFANGSDOSIS VON VITAMIN D VERBINDUNGEN
PROCEDE DE DETERMINATION DE LA DOSE INITIALE DE COMPOSES DE VITAMINE D

(30) Priority: 13.10.2000 US 240126 P
(43) Date of publication of application: 13.08.2003
(60) Divisional application: 06111818.8
(73) Proprietor: ABBOTT LABORATORIES, Abbott Park, IL 60064-6050 (US)
(72) Inventor: AMDAHL, Michael, J., Wauconda, IL 60084 (US)
(74) Representative: Modiano, Micaela Nadia
(86) International application number: PCT/US2001/042708
(87) International publication number: WO 2002/031514

(56) References cited:
- MARTIN K J ET AL: "Therapy of secondary hyperparathyroidism with 19-nor-1alpha,25-dihydroxyvitamin D2." AMERICAN JOURNAL OF KIDNEY DISEASES: THE OFFICIAL JOURNAL OF THE NATIONAL KIDNEY FOUNDATION. UNITED STATES OCT 1998, vol. 32, no. 2 Suppl 2, October 1998 (1998-10), pages S61-S66, XP009005494 ISSN: 0272-6386
- CADA DJ ET AL: "Paricalcitol injection and Ribavirin/Interferon alfa-2b." HOSPITAL PHARMACY, vol. 34, no. 3, 1999, pages 303-338, XP009005495 United States ISSN: 0018-5787
- LLACH F ET AL: "The importance of dosing intravenous calcitriol in dialysis patients with severe hyperparathyroidism." AMERICAN JOURNAL OF KIDNEY DISEASES: THE OFFICIAL JOURNAL OF THE NATIONAL KIDNEY FOUNDATION. UNITED STATES NOV 1995, vol. 26, no. 5, November 1995 (1995-11), pages 845-851, XP009005476 ISSN: 0272-6386
- SPRAGUE S M ET AL: "Safety and efficacy of long-term treatment of secondary hyperparathyroidism by low-dose intravenous calcitriol." AMERICAN JOURNAL OF KIDNEY DISEASES: THE OFFICIAL JOURNAL OF THE NATIONAL KIDNEY FOUNDATION. UNITED STATES JUN 1992, vol. 19, no. 6, June 1992 (1992-06), pages 532-539, XP009005477 ISSN: 0272-6386
- MARTIN K J ET AL: "Paricalcitol dosing according to body weight or severity of hyperparathyroidism: a double-blind, multicenter, randomized study." AMERICAN JOURNAL OF KIDNEY DISEASES: THE OFFICIAL JOURNAL OF THE NATIONAL KIDNEY FOUNDATION. UNITED STATES NOV 2001, vol. 38, no. 5 Suppl 5, November 2001 (2001-11), pages S57-S63, XP009005498 ISSN: 1523-6838

## Description

### Field of the Invention

The present invention is directed to a method for determining the initial dose of vitamin D compounds when used for the treatment of secondary hyperparathyroidism and renal osteodystrophy.

### Background of the Invention

Renal osteodystrophy, which encompasses a host of metabolic and morphologic abnormalities of the bone, is an early complication of kidney disease. Elevation of intact parathyroid hormone (iPTH; used interchangeably with "PTH") secondary to renal failure (also referred to as secondary hyperparathyroidism) is a major contributor to high-turnover renal osteodystrophy. The various disorders of bone formation with high-turnover renal osteodystrophy may be accompanied by such conditions as fractures and bone deformities, bone cysts, osteopenia, resistance to erythropoietin caused by marrow fibrosis, intractable pruritus, spontaneous tendon rupture, periarthritis and joint pain, myopathy, growth failure in children, and extraskeletal calcification.

Through appropriate monitoring and treatment, many patients with secondary hyperparathyroidism from renal failure can maintain mobility and physical function and circumvent the need for surgical parathyroidectomy. Apart from dialysis, such treatment may include the use of vitamin D compounds such as calcitriol and paricalcitol.

Calcitriol (also referred to as 1,25-(OH)₂D₃; 1α,25-(OH)₂D₃; 1,25 dihydroxycholecalciferol or 1,25 dihydroxy vitamin D) is a vitamin D₃ analog and is the metabolically active form of vitamin D. Pericalcitol (also referred to as 19-nor 1,25-(OH)₂D₂ or 19-nor 1,25 dihydroxy vitamin D₂) is a vitamin D₂ derivative. Both compounds suppress PTH levels with minimal effect on calcium and phosphorus levels. These compounds have been approved and marketed in the United States for the prevention and treatment of secondary hyperparathyroidism associated with chronic renal failure in adults. These approvals were based on the results of controlled clinical trials performed in patients with end-stage renal disease (ESRD). The currently approved starting dose of calcariol injection (CALCIJEX® , Abbott Laboratories) is 0.02 microgram/kilogram dry weight (µg/kg) and for paricalcitol injection (ZEMPLAR® , Abbott Laboratories), 0.04 µg/kg.

Currently, the first or starting dose for patients undergoing treatment for secondary hyperparathyroidism is based on the weight of the patient Many pharmatherapies recommend initial drug dosing to be based upon weight These recommendations are derived from the need to reach therapeutic levels based upon the distribution of the medication throughout the patient's body fluid compartment. The approximate amount of this fluid can be determined, based upon the patients body weight However, patients with secondary hyperparathyroidism as a result of ESRD have large fluctuations in body weight due to their inability to eliminate excess fluid via the kidneys. Fluctuations may be as little as 1 kg daily to as much as 20 kg daily. Therefore, attempts to prescribe a starting dose of a vitamin D compound based upon the current weight often prohibits accurate estimation.

Dosing of vitamin D compounds based on a range of PTH provides an alternative to the approach based on weight. However, the published ranges require interpretation by the attending medical professional and do not address patients with severe elevated levels of PTH. Thus, while the published ranges are considered safe they may not be necessarily efficacious.

Martin et al., American Journal of Kidney Diseases (Oct 1998), 32(4), Suppl 2, S61-S66 and Cada et al., Hospital Pharmacy (1999), 34(3), 303-338, disclose studies related to treatment of secondary hyperparathyroidism using paticalcitol with doses were adjusted based on PTH level, serum calcium and calcium-phosphorus product.

Llach et al., American Journal of Kidney Diseases (Nov 1995), 26(5), 845-851, and Sprague et al., American Journal of Kidney Diseases (Jun 1992), 19(6), 532-539, disclose studies related to treatment of patients with hyperparathyroidism using calcitriol with doses based on serum PTH level.

Inaccurate estimations of starting doses for vitamin D therapy may delay effective treatment of secondary hyperparathyroidism. Such delays have been associated with prolonged elevations of PTH and the resultant altered metabolism. These alterations, often resulting in hypercalcemia and/or hyperphosphatemia, put patients at risk for cardiovascular complications.

(Block GA, Port FK, "Re-evaluation of risks associated with hyperphosphatemia and hyperparathyroidism in dialysis patients: recommendations for a change in management", American Journal of Kidney Diseases, June 2000, 35(6): 1226-37, Goodman WG, Goldin J, Kuison BD, Yoon C, Gales B, Sider D, Wang Y, Chung J, Bmerick A, Greaser L, Elashoff RM, Salusky IB, "Coronary-artery calcification in young adults with end-stage renal disease who are undergoing dialysis", N. Engl. J. Med, May 18, 2000, 342(20): 1478-83.) compounds when used to treat renal osteodystrophy when used to treat renal and/or hyperparathyroidism. We have discovered an easy to use method to determine the safe and efficacious starting dose for patients commencing treatment of secondary hyperparathyroidism. This dosing scheme allows a patient to receive an initial dose of vitamin D based on the patient's PTH level as opposed to the current approved method based on body weight.

### Summary of the Invention

One aspect of the present invention provides a method of determining the initial dose of a vitamin D compound. The method utilizes the final dose as a response variable and baseline PTH as a predictor variable. Both variables can be determined from existing data, typically data generated from clinical trials. Regression analysis is performed on the data to generate the initial dose.

### Detailed Description

As used in the specification, the following terms have the meanings indicated:

The term "vitamin D compound" shall refer to any vitamin D compound, including, an analog, derivative, or active metabolite thereof

The term "baseline PTH value" or "bPTH" shall refer to the patient PTH value at the commencement of treatment with the vitamin D compound.

The term "final dose" shall refer to the final dose (in micrograms) of a vitamin D compound that is associated with the first stabile, clinically significant reduction in patient PTH values as determined for the vitamin D compound.

The term "initial dose" shall refer to the dose in micrograms that is the first or starting dose of the vitamin D compound administered to the patient as the patient commences treatment for secondary hyperparathyroidism and/or renal osteodystrophy. Initial dose is equal to the baseline PTH divided by a denominator based upon the outcome of a regression model.

The present invention can be utilized to determine the starting dose of a vitamin D compound when used for the treatment of secondary hyperparathyroidism and/or renal osteodystrophy, Thus, the present invention is suitable for use in determining the initial dose of various vitamin D compounds currently approved for administration into humans, e.g., paricalcitol, calcitriol and doxercalciferol. Most preferred is the use of the present invention in determining the initial dose of paricalcitol.

Once the initial dose is determined for a vitamin D compound, the initial dose can be administered to a patient commencing treatment for renal osteodystrophy and/or secondary hyperparathyroidism with the vitamin D compound. The method described herein can be utilized in the treatment of these conditions regardless of the route of administration of the vitamin D compound. Preferably, when the vitamin D compound is administered according to the method of the invention, the administration is either oral or by injection, more preferably by intravenous injection.

We have determined that the initial dosing of vitamin D compounds can be based on patient baseline PTH while maintaining a safety profile consistent with approved dosing protocols, with no difference in the incidence of hypercalcemia. The method of the present invention utilizes regression analysis, preferably a zero-intercept linear model, to calculate an initial dose for the vitamin D compound, The data used in the model can be derived from a retrospective study of existing data. For example, the initial dose of paricalcitol has been determined from a retrospective study of clinical data. Thus, as long as sufficient dosing data is available to conduct the statistical analysis for a vitamin D compound, the method of the invention can be used to determine the initial dose for any vitamin D compound.

Once the model is in place, the predictability of the model can be verified by comparing the PTH values predicted by the model versus actual PTH values.

The determination of the initial dose is accomplished as follows. As a first step the patient's baseline PTH value is determined prior to the commencement of treatment with the vitamin D compound. Generally, patients having PTH values greater than 200 picograms per milliliter are considered to be candidates for vitamin D therapy. The determination ofPTH values, including baseline PTH, is accomplished using methods that are well known in the art.

In addition to baseline PTH, the final dose must also be determined. Final dose is the amount of vitamin D compound that was administered prior to the first determination of a stabile, clinically significant reduction in PTH values. In practice, the vitamin D compound is administered and PTH values are monitored, generally at least weekly, until such time as the patient's PTH values have been lowered by a clinically significant value and remain stabile at that value. A clinically significant reduction, typically reported as a percent reduction from baseline PTH, is that percent reduction which has been determined to be of clinical significance. The clinical significance of a percent reduction is generally determined in a clinical trial of efficacy for the vitamin D compound and thus can range from about thirty percent to about sixty percent. In the preferred method of the invention, a clinically significant reduction is achieved with a thirty percent reduction in baseline PTH values. In addition to a clinically significant reduction in baseline PTH, the PTH reduction must be stabile for a period of time. The stability of the reduction must also be experimentally determined as it is also dependent on the vitamin D compound that is the subject of the treatment and is typically also determined in a clinical trial of efficacy for the vitamin D compound. In a preferred method of the invention utilizing the vitamin D₂ compound paricalcitol, the PTH value is considered to be stabile when the patient's PTH value shows a thirty percent reduction, which reduction is stabile for at least 28 days. Thus, the final dose would be that dose of paricalcitol that was administered prior to the first stabile reduction in PTH values.

Finally, regression analysis is used to determine initial dose. One such method utilizes zero intercept linear regression. The zero intercept model is preferred for its ease of use by the medical professional as only one task must be completed which minimizes the risk of a mistake being made in the calculation. In this model, final dose is the response variable and baseline PTH is the predictor variable. It will be apparent to those skilled in the art that alternate regression models, e.g., multiple regression analysis, could also be employed to determine the initial dose.

### Examples

### Example 1 - Determination of Initial Dose (Model)

An exploratory analysis of a long-term open label study of paricalcitol injection (ZEMPLAR® , Abbott Laboratories) was performed in an attempt to discover a relatively safe and effective method of determining the starting dose of ZEMPLAR based on a patient's baseline PTH. Those patients who achieved a thirty percent decrease from baseline PTH for at least 28 days (4 weeks) were used in the analysis. The dose associated with the first thirty percent decrease (the final dose) of this PTH reduction period was determined. Using final dose as the response variable and a patient's baseline PTH as the predictor variable, a regression analysis was performed. A zero intercept model was employed so as to allow the physician a relatively easy method for determining the starting dose of the drug. The regression analysis produced the following model for initial dose: initial dose (micrograms) equal to baseline PTH / 80.

The results show that the model will slightly underpredict the starting dose for lower values of baseline PTH. This may be desirable since patients with less significant hyperparathyroidism may benefit from less aggressive therapy.

### Example 2- Safety and Efficacy of Initial Dose

A double-blind, randomized, 12-week trial was conducted in 125 adult ESRD patients to determine if a starting dose of paricalcitol injection using baseline PTH/80 was equivalent in the rate of hypercalcemia (single episode, Ca >11.5 miligram/deciliter) compared to the approved initial dose (0.04 µg/kg, dry weight). Patients were randomized (1:1) to doses by either PTH/80 or 0.04 µg/kg. Baseline demographics and laboratory values were similar between groups. Dosing occurred 3× weekly per patient hemodialysis schedule, Dose increases of 2 µg could occur once per 2 weeks; decreases of 2 µg could occur once per week. Patients completed the study by reducing PTH ≥30% from baseline for 4 consecutive weekly measurements, or by having a single incidence of hypercalcemia, or by completing 12 weeks of treatment. The primary analysis was a comparison of the incidence of hypercalcemia between groups. Secondary analyses included the time in days to the first of 4 consecutive ≥30% decreases from baseline PTH levels, the difference in the number of dose adjustments to achieve the first of 4 consecutive ≥30% decreases from baseline PTH levels, and the difference in the incidence rates of 2 consecutive occurrences of CaxP>75. Results are presented in Table 1.

**Table 1**

| **Parameter** | **PTH/80** | **0.04.µg /kg** |
|---|---|---|
| Incidence(s) of Hypercalcemia | 0 | 0 |
| Median Days to First of 4≥30% PTH Decreases* | 31 | 45 |
| Median Number of Dose Adjustments | 2 | 3 |
| Incidences of Ca x P >75 | 5 | 2 |
| Mean PTH (pg/mL) Decrease (SE) | -259 (24.01) | -193 (24.59) |

| | | |
|---|---|---|
| *Statistically significant (P=0.0306) | | |

The safety profile (adverse events, laboratory results, vital signs) was similar between treatment groups. In conclusion, dosing based on the severity of hyperparathyroidism incurred no additional risk of hypercalcemia and proved a safe, effective, and simple alternative to dosing based on dry weight.

## Claims

1. A method of determining the initial dose of a vitamin D compound, comprising:
a) determining the baseline PTH value (bPTH) of a patient,
b) determining the final dose of the vitamin D compound, where the final dose is that dose of vitamin D compound associated with a first stable clinically significant reduction in patient intact parathyroid hormone (PTH),
c) applying the baseline PTH and final dose to regression analysis.
d) calculating the initial dose of the vitamin D compound,

2. The method of claim 1 wherein the regression analysis is a zero intercept linear model.

3. The method of claim 1 wherein the vitamin D compound is a vitamin D₂ compound.

4. The method of claim 3 wherein the vitamin D₂ compound is paricalcitol.

5. The method of claim 4 wherein the initial dose is bPTH/80.

6. Use of a vitamin D compound for manufacturing a medicament for treating end-stage renal disease (ESRD) in a patient by administration to the patient an initial dose of bPTH/80.

7. Use of a vitamin D compound for manufaturing a medicament for treating secondary hyperparathyroidism, in a patient undergoing vitamin D therapy by administration to the patient, the initial dose of bPTH/80.

## Patentansprüche

1. Ein Verfahren zur Bestimmung der Anfangsdosis einer Vitamin D Verbindung, das folgendes umfaßt:
a) Bestimmen des Basislinien-PTH-Werts (bPTH) eines Patienten,
b) Bestimmen der endgültigen Dosis der Vitamin D Verbindung, wobei die endgültige Dosis diejenige Dosis der Vitamin D Verbindung ist, die mit einer ersten stabilen klinisch signifikanten Reduktion des intakten Parathormons (PTH) eines Patienten in Zusammenhang steht,
c) Unterziehen des Basislinien PTH-Werts und der endgültigen Dosis einer Regressionsanalyse,
d) Berechnen der anfänglichen Dosis der Vitamin D Verbindung.

2. Das Verfahren von Anspruch 1, worin die Regressionsanalyse ein lineares Null-Schnittpunkt Modell ist.

3. Das Verfahren von Anspruch 1, worin die Vitamin D Verbindung eine Vitamin D₂ Verbindung ist.

4. Das Verfahren von Anspruch 3, worin die Vitamin D₂ Verbindung Paricalcitol ist.

5. Das Verfahren von Anspruch 4, worin die Anfangsdosis bPTH/80 ist.

6. Verwendung einer Vitamin D Verbindung zur Herstellung eines Medikaments zur Behandlung einer Nierenerkrankung im Endstadium (end-stage renal disease, ESRD) in einem Patienten durch Verabreichung einer anfänglichen Dosis von bPTH/80 an den Patienten.

7. Verwendung einer Vitamin D Verbindung zur Herstellung eines Medikaments zur Behandlung von sekundärem Hyperparathyroidismus in einem Patienten, der einer Vitamin D Therapie unterzogen wird, durch Verabreichen der anfänglichen Dosis von bPTH/80 an den Patienten.

## Revendications

1. Procédé de détermination de la dose initiale d'un composé de vitamine D, comprenant les étapes consistant à :
a) déterminer la valeur de PTH de base (bPTH) d'un patient
b) déterminer la dose finale du composé de vitamine D, la dose finale étant la dose du composé de vitamine D étant associée à une première réduction stable cliniquement significative de l'hormone parathyroïdienne (PTH) intacte du patient
c) appliquer la PTH de base et la dose finale à l'analyse de régression,
d) calculer la dose initiale du composé de vitamine D.

2. Procédé selon la revendication 1 dans lequel l'analyse de régression est un modèle linéaire à point d'intersection zéro.

3. Procédé selon la revendication 1 dans lequel le composé de vitamine D est un composé de vitamine D₂.

4. Procédé selon la revendication 3 dans lequel le composé de vitamine D₂ est le paricalcitol.

5. Procédé selon la revendication 4 dans lequel la dose initiale est bPTH/80.

6. Utilisation d'un composé de vitamine D pour fabriquer un médicament pour traiter l'insuffisance rénale terminale (IRT) chez un patient par administration au patient d'une dose initiale de bPTH/80.

7. Utilisation d'un composé de vitamine D pour fabriquer un médicament pour traiter l'hyperparathyroïdie secondaire chez un patient suivant un traitement par la vitamine D par administration au patient de la dose initiale de bPTH/80.
